(19) Europäisches Patentamt
European Patent Office
Office européen des brevets

(11) Veröffentlichungsnummer: **0 315 017**
**A2**

(12) # EUROPÄISCHE PATENTANMELDUNG

(21) Anmeldenummer: **88117701.8**

(51) Int. Cl.⁴: **A61K 31/445 , A61K 31/70**

(22) Anmeldetag: **25.10.88**

(30) Priorität: **05.11.87 DE 3737523**

(43) Veröffentlichungstag der Anmeldung:
**10.05.89 Patentblatt 89/19**

(84) Benannte Vertragsstaaten:
**AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Böshagen, Horst, Dr.**
**Wiesenstrasse 4**
**D-5657 Haan(DE)**
Erfinder: **Junge, Bodo, Dr.**
**Spiekern 23**
**D-5600 Wuppertal 23(DE)**
Erfinder: **Kinast, Günther, Dr.**
**Am Eckbusch 15 A**
**D-5600 Wuppertal 1(DE)**
Erfinder: **Schüller, Matthias, Dr. Dr.**
**Gellertweg 20**
**D-5600 Wuppertal 1(DE)**
Erfinder: **Stoltefuss, Jürgen**
**Parkstrasse 20**
**D-5657 Haan(DE)**
Erfinder: **Paessens, Arnold, Dr.**
**Stresemannstrasse 56**
**D-5657 Haan(DE)**

(54) **Verwendung von 2-Hydroxymethylen-3,4,5-trihydroxypiperidinen als antivirale Mittel.**

(57) Die Erfindung betrifft die Verwendung von bekannten, substituierten Hydroxypiperidinen, als antivirale Mittel in der Human- und Tiermedizin.

**EP 0 315 017 A2**

Xerox Copy Centre

## Verwendung von 2-Hydroxymethylen-3,4,5-trihydroxypiperidinen als antivirale Mittel

Die Erfindung betrifft die Verwendung von bekannten 2-Hydroxymethylen-3,4,5-Trihydroxypiperidinen als antivirale Mittel in der Human- und Tier-Medizin.

Es ist bekannt, daß Desoxynojirimycin-Derivate Glukosidaseinhibitoren sind EP 947; EP 8058; EP 34 784; DE 2 848 117. Darüberhinaus ist bekannt, daß einige Desoxynojirimycin-Derivate herbizide Wirkungen haben DE 3 024 901.

Es wurde nun gefunden, daß 2-Hydroxymethylen-3,4,5-trihydroxypiperidine der allgemeinen Formel (I)

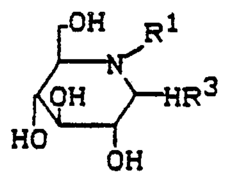

(I)

in welcher

$R^1$ für Wasserstoff steht und

$R^3$ für einen gegebenenfalls substituierten geradkettigen, verzweigten oder cyclischen gesättigten oder ungesättigten, gegebenenfalls durch Heteroatome wie O oder S, unterbrochenen Kohlenwasserstoffrest, einen gegebenenfalls substituierten aromatischen Rest oder einen gegebenenfalls substituierten heterocyclischen Rest steht, gute antivirale Wirkung haben.

Bevorzugt bedeutet $R^3$ $C_1$-$C_{30}$-Alkyl, $C_2$-$C_{18}$-Alkenyl, $C_2$-$C_{18}$-Alkinyl, $C_3$-$C_{12}$-Cycloalkyl, -Cycloalkenyl, -Cycloalkadienyl, -Bicycloalkyl, -Bicycloalkenyl, -Bicycloalkadienyl, -Tricycloalkyl, -Tricycloalkenyl oder -Tricycloalkadienyl, Phenyl, Naphthyl, $C_3$-$C_7$-Heterocyclyl mit 1 bis 4 Heteroatomen aus der Reihe N, O oder S, an den ein Benzolrest ankondensiert sein kann, wobei die aufgeführten Reste 1 bis 5 Substituenten tragen können.

Als Substituenten von Phenyl, Naphthyl und Heterocyclyl kommen Halogen, insbesondere Chlor, Brom und Fluor, $C_1$-$C_4$-Alkyl, Halogen-$C_1$-$C_4$-alkyl, $C_1$-$C_4$-Alkoxy, $C_1$-$C_4$-Alkylmercapto, $C_1$-$C_4$-Alkylsulfonyl, Nitro, Cyano, Di-$C_1$-$C_{12}$-alkylamino, Di-$C_1$-$C_{12}$-alkylaminosulfonyl, Di-$C_1$-$C_{12}$-dialkylaminocarbonyl, Pyrrolidino, Pyrrolidinosulfonyl, Pyrrolidinocarbonyl, Piperidino, Piperidinosulfonyl, Piperidinocarbonyl, Morpholino, Morpholinosulfonyl, Morpholinocarbonyl, N′-$C_1$-$C_4$-alkylpiperazino, N′-$C_1$-$C_4$-alkylpiperazinosulfonyl, N′-$C_1$-$C_4$-alkylpiperazinocarbonyl, Pyridyl, Thienyl, Imidazolyl, Isoxazolyl, Thiazolyl, Glucopyranosyl und Ribofuranosyl in Frage.

Als Substituenten der übrigen Definitionen von $R^3$ seien beispielhaft genannt $C_1$-$C_4$-Alkyl, $C_1$-$C_4$-Alkoxy, $C_1$-$C_4$-Alkylmercapto, Halogen, insbesondere Fluor, Chlor und Brom, Di-$C_1$-$C_{12}$-alkylamino, Pyrrolidino, Piperidino, Morpholino, N′-$C_1$-$C_4$-alkylpiperazino, gegebenenfalls durch Halogen, insbesondere Fluor, Chlor und Brom, $C_1$-$C_4$-Alkyl, $C_1$-$C_4$-Alkoxy, $C_1$-$C_4$-Alkylmercapto, $C_1$-$C_4$-Alkylsulfonyl, Nitro, $C_1$-$C_4$-Alkoxycarbonyl, Di-$C_1$-$C_{12}$ alkylaminocarbonyl, Di-$C_1$-$C_{12}$-alkylaminosulfonyl, Pyridyl, Thienyl, Imidazolyl, Isoxazolyl, Thiazolyl, Glucopyranosyl und Ribofuranosyl substituiertes Phenyl oder Naphthyl.

Die Alkylreste $R^3$ konnen darüber hinaus $C_3$-$C_{12}$-Cycloalkyl, -Cycloalkenyl, -Cycloalkadienyl, -Bicycloalkyl, -Bicycloalkenyl, -Bicycloalkadienyl, -Tricycloalkyl, -Tricycloalkenyl oder -Tricycloalkadienyl als Substituenten tragen.

In bevorzugten Verbindungen der Formel (I) bedeutet $R^1$ $C_1$-$C_{18}$-Alkyl, gegebenenfalls durch 1 bis 5 Halogenatome substituiert, $C_3$-$C_{10}$-Alkenyl, $C_3$-$C_{10}$-Alkinyl, $C_3$-$C_7$Cycloalkyl oder gegebenenfalls durch Halogen, $C_1$-$C_4$-Alkyl, $C_1$-$C_4$-Alkoxy oder Nitro substituiertes Phenyl.

Bekannt sind diese Verbindungen aus DE 2 848 117.

Ebenfalls eine gute antivirale Wirkung haben Verbindungen der allgemeinen Formel (I)

in welcher

$R^1$ für Wasserstoff oder für einen gegebenenfalls substituierten, geradkettigen, verzweigten oder cyclischen, gesättigten oder ungesättigten Kohlenwasserstoffrest, oder für einen gegebenenfalls substituierten aromatischen oder heterocyclischen Rest steht

und

$R^3$ für -H, OH, -$OR^5$, -SH, $SR^5$, -$NH_2$, -$NHR^5$,

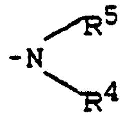
,

$NH_2CH_2$-, $NHR^5$-$CH_2$-, $NR^5R^4$-$CH_2$-, -COOH, -$COOR^5$, HO-$CH_2$-, $R^5$CO-$NHCH_2$-, $R^5$CO-$NR^4CH_2$-, $R^5SO_2NHCH_2$-, $R^5SO_2$-$NR^4CH_2$-, $R^5$-NH- $\underset{\overset{\|}{O}}{C}$ -NH-$CH_2$-,

$R^5$-NH- $\underset{\overset{\|}{S}}{C}$ -NH-$CH_2$-,

$R^5$-O- $\underset{\overset{\|}{O}}{C}$ -NH-$CH_2$-,

-$SO_3H$, -CN, -$CONH_2$, $CONHR^5$ oder $CONR^5R^4$ steht,
wobei
$R^5$, $R^4$ die vorstehend für $R^1$ genannten Bedeutungen annehmen konnen,
und wobei für den Fall $R^3$=H oder OH, $R^1$ für einen gegebenenfalls substituierten, geradkettigen, verzweigten oder cyclischen, gesättigten oder ungesättigten Kohlenwasserstoffrest oder für einen gegebenenfalls substituierten aromatischen oder heterocyclischen Rest steht (d.h. daß $R^1$ nicht H ist).

Bevorzugt bedeuten $R^4$, $R^5$ und $R^4$ einen Alkylrest mit 1 bis 30, insbesondere 1 bis 18 C-Atomen; einen Alkenylrest oder Alkinylrest mit 2 bis 18, insbesondere 3 bis 10 C-Atomen; einen mono-, bi- oder tricyclischen Rest mit 3 bis 10 C-Atomen, der gesättigt oder einfach oder doppelt ungesättigt sein kann, einen Arylrest mit 6 bis 10 C-Atomen, einen heterocyclischen Rest mit 3 bis 8 insbesondere 3 bis 6 Ringgliedern, der 1, 2, 3 oder 4 Heteroatome, insbesondere N, O, S enthalten kann und an dem ein Benzolring oder ein weiterer Heterozyklus der genannten Art ankondensiert sein kann, wobei die genannten Reste 1 bis 5, insbesondere 1, 2 oder 3 Substituenten tragen können.

Als Substituenten für Alkyl seien beispielhaft aufgeführt: Hydroxy oder Alkoxy mit vorzugsweise 1 bis 4 Kohlenstoffatomen, insbesondere Methoxy und Ethoxy; Acyloxy, wobei der Acylrest von aliphatischen Carbonsäuren mit 1 bis 7 C-Atomen, aromatischen Carbonsäuren insbesondere Phenylcarbonsäuren, die im Phenylrest durch -OH, -Halogen, insbesondere F, Cl, Br, $C_1$-$C_4$-Alkyl, $C_1$-$C_4$-Alkoxy, Nitro und/oder Amino substituiert sein können, heterocyclischen Carbonsäuren, die sich von 5-oder 6-gliedrigen Heterocyclen ableiten, die 1 bis 3 Heteroatome (N, O, S) enthalten und im heterocyclischen Ring durch $C_1$-$C_4$-Alkyl, Chlor, Brom, Amino substituiert sein können, abgeleitet ist; Amino, Monoalkylamino und Dialkylamino mit vorzugsweise 1 bis 4 Kohlenstoffatomen je Alkylrest, insbesondere Monomethylamino, Monoethylamino, Dimethylamino und Diethylamino, Monoacylamino, wobei der Acylrest von aliphatischen Carbonsäuren mit 1 bis 7 C-Atomen, aromatischen Carbonsäuren, insbesondere Phenylcarbonsäuren, die im Phenylrest durch -OH, -Halogen, insbesondere F, Cl, Br, $C_1$-$C_4$-Alkyl, $C_1$-$C_4$-Alkoxy, Nitro und/oder Amino substituiert sein können, heterocyclischen Carbonsäuren, die sich von 5- oder 6-gliedrigen Heterocyclen ableiten, die 1 bis 3 Heteroatome (N, O, S) enthalten und im heterocyclischen Ring durch $C_1$-$C_4$-Alkyl, Chlor, Brom, Amino substituiert sein können, abgeleitet ist.

Mercapto, Alkylthio mit vorzugsweise 1 bis 4 Kohlenstoffatomen, insbesondere Methylthio und Ethylthio; Halogen, vorzugsweise Fluor, Chlor und Brom; Alkylcar bonyl mit vorzugsweise 1 bis 4 Kohlenstoffatomen im Alkylrest; Carboxy, Nitro, Cyan, die Aldehydfunktion, die Sulfonsäuregruppe; sowie heterocyclische Reste der oben genannten Art, insbesondere auch von Zuckern, ganz besonders von Hexosen oder Pentosen abgeleitete heterocyclische Reste, die direkt über ein Ringatom oder über eine -O-, -S- oder -NH-Brücke mit dem Alkylrest verbunden sein können,

Beispiele für heterocyclische Substituenten der Alkylreste sind: Phthalimido, Pyridyl, Thienyl, Furyl, Isoxazolyl, Thiazolyl, Glucopyranosyl, Ribofuranosyl, Oxiranyl u. dgl.
Desweiteren eignen sich als Substituenten der Alkylreste aromatische Reste wie Naphthyl und insbesondere Phenyl, die einen oder mehrere, vorzugsweise 1 bis 3 gleiche oder verschiedene Substituenten aus der Reihe -OH, -$NH_2$, $C_1$-$C_4$-Alkyl-NH-, $C_1$-$C_4$-Dialkyl-N-, $C_1$-$C_4$-Alkoxy, $NO_2$-, -CN, -COOH, -COO-Alkyl ($C_1$-$C_4$), $C_1$-$C_6$-Alkyl, Halogen, insbesondere Fluor, Chlor oder Brom, $C_1$-$C_4$-Alkylthio, -SH, $C_1$-$C_4$-Alkylsulfonyl, -$SO_3H$, -$SO_2$-$NH_2$, -$SO_2$-NH-Alkyl ($C_1$-$C_4$) tragen können.

Der Alkylrest kann auch einen mono-, bi- oder tricyclischen Substituenten mit vorzugsweise 3 bis 10 Kohlenstoffatomen tragen, der seinerseits durch Hydroxy, Amino, Halogen, insbesondere Fluor, Chlor, Brom, oder -COOH substituiert sein kann.

Der Alkylrest trägt bevorzugt Substituenten wie Hydroxy oder Alkoxy mit 1 bis 4 Kohlenstoffatomen; Mercapto oder Alkylthio mit 1 bis 4 Kohlenstoffatomen, Halogen, Nitro, Amino, Monoalkylamino mit 1 bis 4

C-Atomen und Acylamino, wobei der Acylrest von aliphatischen Carbonsäuren mit 1 bis 6 C-Atomen abgeleitet ist.

Für die cyclischen mono-, bi- oder tricyclischen Reste $R^1$, $R^5$ und $R^4$ kommen die für die Alkylreste genannten Substituenten in Betracht.

Die Arylreste können einen oder mehrere, vorzugsweise 1 bis 3 gleiche oder verschiedene Substituenten tragen. Als Substituenten seien beispielhaft aufgeführt: Alkyl mit 1 bis 10 C-Atomen, das seinerseits wieder beispielsweise durch Chlor, Nitro oder Cyan substituiert sein kann; gegebenenfalls substituierte Alkenylreste mit 1 bis 10 Kohlenstoffatomen; Hydroxy, Alkoxy mit vorzugsweise 1 bis 4 Kohlenstoffatomen; Amino, Monoalkyl- und Dialkylamino mit vorzugsweise 1 bis 4 Kohlenstoffatomen je Alkylrest; Mercapto, Alkylthio mit vorzugsweise 1 bis Kohlenstoffatomen; Carboxy, Carbalkoxy mit vorzugsweise 1 bis 4 Kohlenstoffatomen; die Sulfonsäuregruppe, Alkylsulfonyl mit vorzugsweise 1 bis 4 Kohlenstoffatomen, Arylsulfonyl, vorzugsweise Phenylsulfonyl; Aminosulfonyl Alkylamino- und Dialkylaminosulfonyl mit 1 bis 4 Kohlenstoffatomen je Alkylgruppe, vorzugsweise Methyl- und Dimethylaminosulfonyl; Nitro, Cyan oder die Aldehydgruppe, Alkylcarbonylamino mit vorzugsweise 1 bis 4 Kohlenstoffatomen; Alkylcarbonyl mit 1 bis 4 Kohlenstoffatomen; Benzoyl, Benzylcarbonyl und Phenylethylcarbonyl, wobei die zuletzt genannten Alkyl, Phenyl, Benzyl und Phenylethylreste ihrerseits wieder beispielsweise durch Chlor, Nitro oder Hydroxy substituiert sein können.

Die heterocyclischen Reste $R^1$ sind bevorzugt von heteroparaffinischen, heteroaromatischen oder heteroolefinischen 5- oder 6-gliedrigen Ringen mit vorzugsweise 1 bis 3 gleichen oder verschiedenen Heteroatomen abgeleitet. Als Heteroatome stehen Sauerstoff, Schwefel oder Stickstoff. Diese Ringsysteme können weitere Substituenten wie beispielsweise Hydroxy-, Amino- oder $C_1$-$C_4$-Alkylgruppen tragen oder an sie können Benzolkerne oder weitere vorzugsweise 6-gliedrige heterocyclische Ring der genannten Art annelliert sein.

Besonders bevorzugte heterocyclische Reste leiten sich beispielsweise von Furan, Pyran, Pyrrolidin, Piperidin, Pyrazol, Imidazol, Pyrimidin, Pyridazin, Triazin, Pyrrol, Pyridin, Benzimidazol, Chinolin, Isochinolin oder Purin ab.

In den Verbindungen der Formel I steht $R^3$ vorzugsweise für -H -OH, $-SO_3H$, -CN, $-CH_2NH_2$, $-CH_2NH-(C_1-C_{14}\text{-Alkyl})$, $-CH_2-NH-\underset{\underset{O}{\|}}{C}-(C_1-C_{14}\text{-Alkyl})$,

$-CH_2-NH-SO_2-(C_1$ bis $C_{14})$-Alkyl, $-CH_2-NH-SO_2$-Phenyl, $R^5-NH-C-NH-CH_2-R^5-NH-\underset{\underset{S}{\|}}{C}-NH-CH_2-$ oder $R^5-O-\underset{\underset{O}{\|}}{C}-NH-CH_2-$.

Ganz besonders bevorzugt steht $R^3$ für -H, $-SO_3H$, -CH, $R^5-NH-\underset{\underset{O}{\|}}{C}-NH-CH_2-$.

Bekannt sind diese Verbindungen aus EP 947.

Weiterhin zu nennen sind Verbindungen der allgemeinen Formel (I) in denen
$R^1$ für

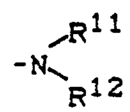

steht
worin
$R^{11}$ Wasserstoff, -Formyl, $-R^{14}$, $-COR^{14}$, $CO_2R^{14}$, $CONH_2$, $CONHR^{14}$, $CONR^{14}R^{15}$, $CSR^{14}$, $CSNH_2$, $CSNHR^{14}$, $CSNR^{14}R^{15}$, $SO_3H$ oder $SO_2R^{14}$,
$R^{12}$ Wasserstoff oder $R^{14}$ oder
$R^{11}$ und $R^{12}$ gemeinsam die Gruppierung

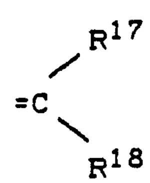

$R^3$ Wasserstoff, Hydroxy, $OR^{14}$, Mercapto, $SR^{14}$, Amino, $NHR^{14}$, $NR^{14}R^{15}$, Cyano, Carboxy, $CO_2R^{14}$, Carboxamido, $CONHR^{14}$, $CONR^{14}R^{15}$, Aminomethyl, $CH_2NHR^{14}$, $CH_2NR^{14}R^{15}$, $CH_2$-$NR^{14}COR^{15}$, $CH_2NR^{14}SO_2R^{15}$, Sulfo, Hydroxymethyl, $CH_2OR^{14}$ oder $CH_2OCOR^{14}$, bedeuten, und

$R^{14}$, $R^{15}$ unabhängig voneinander einen gegebenenfalls substituierten, geradkettigen, verzweigten oder cyclischen gesättigten oder ungesättigten aliphatischen Kohlenwasserstoffrest oder einen gegebenenfalls substituierten aromatischen oder heterocyclischen Rest, wobei 2 Reste $R^{15}$, $R^{17}$ und $R^{18}$ auch miteinander verknüpft sein können, bedeuten, und

$R^{17}$ und $R^{18}$ unabhängig voneinander Wasserstoff oder $R^{15}$ bedeuten.

Bevorzugt bedeuten $R^{14}$, $R^{15}$ Alkyl mit 1 bis 30, insbesondere 1 bis 18 C-Atome, Alkenyl oder Alkinyl mit 2 bis 18, insbesondere 3 bis 10 C-Atomen, Mono-, Bi- oder Tricyclo-alkyl; -alkenyl oder -alkadienyl mit 3 bis 10 C-Atomen, Aryl mit 6 bis 10 C-Atomen, einen heterocyclischen Rest mit 3 bis 8, insbesondere 3 bis 6 Ringgliedern, der 1, 2, 3 oder 4 Heteroatome, insbesondere N, O oder S enthalten kann und an dem ein Benzolring oder ein weiterer Heterozyklus der genannten Art ankondensiert sein kann, wobei die genannten Reste 1 bis 5, insbesondere 1, 2 oder 3 Substituenten tragen können.

Als Substituenten für Alkyl seien beispielhaft genannt: Hydroxy, Alkoxy mit vorzugsweise 1 bis 4 Kohlenstoffatomen, insbesondere Methoxy und Ethoxy; Alkylcarbonyloxy mit 1 bis 7 C-Atomen; gegebenenfalls durch -OH, -Halogen, insbesondere F, Cl, Br, $C_1$-$C_4$-Alkyl, $C_1$-$C_4$-Alkoxy,

Nitro und/oder Amino substituiertes Benzoyloxy, Heterocyclyl, Carbonyloxy, die sich von 5- oder 6-gliedrigen Heterocyclen ableiten, die 1 bis 3 Heteroatome (N,O,S) enthalten und im heterocyclischen Ring durch $C_1$-$C_4$-Alkyl, Chlor, Brom, Amino substituiert sein können; Amino, Monoalkylamino und Dialkylamino mit vorzugsweise 1 bis 4 Kohlenstoffatomen je Alkylrest, insbesondere Monomethylamino, Monoethylamino, Dimethylamino und Diethylamino; Monoacylamino, wobei der Acylrest von aliphatischen Carbonsäuren mit 1 bis 7 C-Atomen, aromatischen Carbonsäuren, insbesondere Phenylcarbonsäuren, die im Phenylrest durch -OH, -Halogen, insbesondere F, Cl, Br, $C_1$-$C_4$-Alkyl, $C_1$-$C_4$-Alkoxy, Nitro und/oder Amino substituiert sein können, abgeleitet ist; heterocyclischen Carbonsäuren, die sich von 5- oder 6-gliedrigen Heterocyclen ableiten, die 1 bis 3 Heteroatome (N, O, S) enthalten und im heterocyclischen Ring durch $C_1$-$C_4$-Alkyl, Chlor, Brom, Amino substituiert sein können; Mercapto, Alkylthio mit vorzugsweise 1 bis 4 Kohlenstoffatomen, insbesondere Methylthio und Ethylthio; Halogen, vorzugsweise Fluor, Chlor und Brom; Alkylcarbonyl mit vorzugsweise 1 bis 4 Kohlenstoffatomen im Alkylrest; Carboxy, Nitro, Cyan, Formyl, Sulfo; heterocyclische Reste der oben genannten Art, und Reste von Zuckern, insbesondere von Hexosen oder Pentosen abgeleitete heterocyclische Reste, die direkt über ein Ringatom oder über eine -O-, -S- oder -NH-Brücke mit dem Alkylrest verbunden sein können.

Beispiele für die vorstehend genannten Heterocyclen sind Phthalimido, Pyridyl, Thienyl, Furyl, Isoxazolyl, Thiazolyl, Glucopyranosyl, Ribofuranosyl, Oxiranyl.

Der Alkylrest kann auch einen mono-, bi- oder tricyclischen Substituenten mit vorzugsweise 3 bis 10 Kohlenstoffatomen tragen, der seinerseits durch Hydroxy, Amino, Halogen, insbesondere Fluor, Chlor, Brom oder -COOH substituiert sein kann.

Der Alkylrest trägt bevorzugt Substituenten wie Hydroxy, Alkoxy mit 1 bis 4 Kohlenstoffatomen, Mercapto, Alkylthio mit 1 bis 4 Kohlenstoffatomen, Halogen, Nitro, Amino, Monoalkylamino mit 1 bis 4 C-Atomen und Acylamino, wobei der Acylrest von aliphatischen Carbonsäuren mit 1 bis 6 C-Atomen abgeleitet ist.

Für die cyclischen mono-, bi- oder tricyclischen Reste $R^1$ kommen die für die Alkylreste genannten Substituenten in Betracht.

Die Arylreste $R^{14}$, $R^{15}$ können einen oder mehrere, vorzugsweise 1 bis 3, gleiche oder verschiedene Substituenten tragen.

Als Substituenten seien beispielhaft aufgeführt:

Alkyl mit 1 bis 10 C-Atomen, das seinerseits wieder beispielsweise durch Chlor, Nitro oder Cyan substituiert sein kann; gegebenenfalls substituierte Alkenylreste mit 2 bis 10 Kohlenstoffatomen; Hydroxy, Alkoxy mit vorzugsweise 1 bis 4 Kohlenstoffatomen; Amino, Monoalkyl- und Dialkylamino mit vorzugsweise 1 bis 4 Kohlenstoffatomen je Alkylrest; Mercapto, Alkylthio mit vorzugsweise 1 bis 4 Kohlenstoffatomen; Carboxy, Carbalkoxy mit vorzugsweise 1 bis 4 Kohlenstoffatomen, die Sulfonsäuregruppe, Alkylsulfonyl mit vorzugsweise 1 bis 4 Kohlenstoffatomen, Arylsulfonyl, vorzugsweise Phenylsulfonyl, Aminosulfonyl, Alkylamino- und Dialkylaminosulfonyl mit 1 bis 4 Kohlenstoffatomen je Alkylgruppe, vorzugsweise Methyl- und Dimethylaminosulfonyl; Nitro, Cyan, Formyl, Alkylcarbonylamino mit vorzugsweise 1 bis 4 Kohlenstoffatomen; Alkylcarbonyl mit 1 bis 4 Kohlenstoffatomen, Benzoyl, Benzylcarbonyl und Phenylethylcarbonyl, wobei die zuletzt genannten Alkyl-, Phenyl-, Benzyl- und Phenylethylreste ihrerseits wieder beispielsweise durch Chlor, Nitro oder Hydroxy substituiert sein können, Dabei ist Aryl vorzugsweise Phenyl und Naphthyl.

Die heterocyclischen Reste $R^{14}$, $R^{15}$ sind bevorzugt von heteroparaffinischen, heteroaromatischen oder

heteroolefinischen 5- oder 6-gliedrigen Ringen mit vorzugsweise 1 bis 3 gleichen oder verschiedenen Heteroatomen aus der Reihe Sauerstoff, Schwefel oder Stickstoff abgeleitet. Diese Ringsysteme können weitere Substituenten wie Hydroxy-, Amino- oder $C_1$-$C_4$-Alkylgruppen tragen oder an sie können Benzolkerne oder weitere vorzugsweise 6-gliedrige heterocyclische Ringe der genannten Art anelliert sein.

Besonders bevorzugte heterocyclische Reste leiten sich von Furan, Pyran, Pyrrolidin, Piperidin, Pyrazol, Imidazol, Pyrimidin, Pyridazin, Pyrazin, Triazin, Pyrrol, Pyridin, Benzimidazol, Chinolin, Isochinolin und Purin ab.

Für bevorzugte Verbindungen innerhalb der Formel (I) steht $R^3$ für Wasserstoff, Hydroxy, Sulfo, Cyano, Aminomethyl, $C_1$-$C_6$-Alkylaminomethyl und $C_1$-$C_6$-Alkylcarbonylaminomethyl. $R^4$ bedeutet vorzugsweise Hydroxymethyl, Hydroxy-($C_1$-$C_6$-Alkyl)-methyl, $C_1$-$C_7$-Alkyl und $C_1$-$C_5$-Alkoxymethyl.

Ganz besonders bevorzugt seien Verbindungen der Formel (I) genannt, bei denen
$R^1$ für

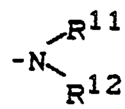

steht,
worin
$R^{11}$ Wasserstoff, $C_1$-$C_4$-Alkyl, $C_1$-$C_4$-Alkoxy, $C_1$-$C_4$-Alkylsulfonyl, $C_1$-$C_4$-Alkylcarbonyl oder -phenyl, insbesondere Wasserstoff, Methyl, Ethyl, Acetyl, Methylsulfonyl, Propionyl oder Phenyl,
$R^{12}$ Wasserstoff, gegebenenfalls durch Hydroxy, $C_1$-$C_4$-Alkoxy, Carboxy, $C_5$-$C_7$-Cycloalkyl oder Phenylsulfonylamino substituiertes $C_1$-$C_{12}$-Alkyl, $C_2$-$C_4$-Alkenyl, gegebenenfalls im Phenylring durch Halogen, insbesondere Fluor oder Chlor, $C_1$-$C_4$-Alkyl, Hydroxy, Di-$C_1$-$C_4$-Alkylamino, $C_1$-$C_4$-Alkoxy substituiertes Phenyl, Phenyl-$C_1$-$C_4$-alkyl oder Benzoylmethyl, $C_5$-$C_7$-Cycloalkyl, Furylmethyl, Pyridylmethyl oder Diphenylmethyl
oder $R^1$ und $R^2$ gemeinsam eine Gruppierung

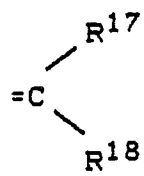

worin
$R^{18}$ Wasserstoff, gegebenenfalls durch Hydroxy, $C_1$-$C_4$-Alkoxy oder Phenylsulfonylamino substituiertes $C_1$-$C_{12}$-Alkyl, $C_5$-$C_7$-Cycloalkyl, $C_2$-$C_{12}$-Alkenyl, Carboxy, gegebenenfalls durch Halogen, insbesondere Fluor und Chlor, $C_1$-$C_4$-Alkyl, Hydroxy, Nitro, Di-$C_1$-$C_4$-alkylamino, $C_1$-$C_4$-Alkoxy oder -carboxy, substituiertes Phenyl, Phenyl-$C_1$-$C_4$-Alkyl oder -benzoyl, Furyl, Pyridyl oder zusammen mit $R_7$ und dem doppelt gebundenen C-Atom einen Cyclopentan- oder Cyclohexan-Ring bedeuten
und $R^{17}$ die Bedeutung von $R^{11}$ hat, und
$R^3$ Wasserstoff, $C_1$-$C_6$-Alkylcarbonylaminomethyl, Phenylcarbonylaminomethyl, Phenylsulfonylaminomethyl, $C_1$-$C_4$-Alkoxycarbonyl und $C_1$-$C_6$-Alkylaminocarbonyl bedeutet.

Ganz besonders bevorzugt seien folgende Wirkstoffe genannt:

Diese Verbindungen sind z.B. aus der Europäischen Patentanmeldung 8 058 bekannt.

Ebenfalls antivirale Wirkung zeigten Verbindungen der allgemeinen Formel (I), in der $R^1$ für

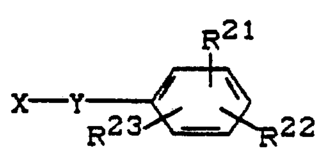

steht,
worin
X für einen geradkettigen oder verzweigten gesättigten oder ungesättigten Kohlenwasserstoffrest steht,
Y für Sauerstoff oder Schwefel steht und
$R^{21}$, $R^{22}$, $R^{23}$ gleich oder verschieden sind und unabhangig voneinander für Wasserstoff, Halogen, Alkyl,

Aryl, Alkoxy, Aroxy, Alkylthio, Nitro, Amino, Hydroxy, Cyano, Alkyl- und Dialkylamino, Acylamino, Acyloxy, Carboxyl, Carbalkoxy, Alkylcarbonyl, Formyl oder einen gegebenenfalls substituierten Sulfonamidrest stehen.

Die Erfindung betrifft vorzugsweise solche Verbindungen der Formel (I), in denen X in $R^1$ einen gesättigten oder ein-oder mehrfach ungesättigten Alkylrest mit 2 bis 10, vorzugsweise 2 bis 5 C-Atomen bedeutet und Y, $R^{21}$, $R^{22}$ und $R^{23}$ die oben angegebene Bedeutung haben.

$R^{21}$, $R^{22}$ und $R^{23}$ bedeuten bevorzugt Wasserstoff, Alkyl, Alkoxy oder Halogen.

Bekannt sind diese Verbindungen aus der DE 3 007 078.

Ganz besonders bevorzugt seien folgende Wirkstoffe genannt:

N-β-Phenoxyethyl-1-desoxynojirimycin
N-(5-Phenoxy-pentyl )-1-desoxynojirimycin
N-(4-Phenoxy-butyl )-1-desoxynojirimycin
N-[β-(2,6-Dimethyl-phenoxy)-ethyl]-1-desoxynojirimycin
N-[γ-(2,6-Dimethoxyphenoxy)-propyl]-1-desoxynojirimycin
N-[β-(2,4-Dichlorphenoxy)-ethyl]-1-desoxynojirimycin
N-(γ-Phenoxypropyl)-1-desoxynojirimycin
N-(4-Phenoxy-trans-buten-2-yl)-1-desoxynojirimycinhydrat
N-(p-Methoxyphenyloxy-trans-buten-2-yl)-1-desoxynojirimycin N-[(4-Carbethoxyphenyloxy)-buten-2-yl]-1-desoxynojirimycin
N-[β-(4-Methoxy-phenoxy)-ethyl]-1-desoxynojirimycin
N-[β-(4-Chlorphenoxy)-ethyl]-1-desoxynojirimycin
N-[β-(4-Cyano-phenoxy)-ethyl]-1-desoxynojirimycin
H-[β-(3-Methylphenoxy)-ethyl]-1-desoxynojirimycin
N-(β-Phenylthioethyl)-1-desoxynojirimycin
N-[β-(4-Methylphenylthio)-ethyl]-1-desoxynojirimycin
N-[4-(4-Methylphenylthio)-buten-2-yl]-1-desoxynojirimycin
N-[4-(4-Chlorphenylthio)-buten-2-yl]-1-desoxynojirimycin
N-[4-(4-tert.-Butylphenylthio)-buten-2-yl] -1-desoxynojirimycin
N-[4-(4-Methylphenylthio)-buten-2-yl] -1-desoxynojirimycin
N-[4-(4-Phenylphenoxy)-buten-2-yl]-1-desoxynojirimycin
N-[β-(4-Acetamidophenoxy)-ethyl]-1-desoxynojirimycin
N-β-(4-Ethoxycarbonyl-phenoxy)-ethyl] -1-desoxynojirimycin
N-[β-(4-Formylphenoxy)-ethyl]-1-desoxynojirimycin
N-[β-(4-Hydroxyphenoxy)-ethyl]-1-desoxynojirimycin
N-[β-(3-Ethoxycarbonylphenoxy)-ethyl] -1-desoxynojirimycin
N-[4-(4-Acetamidophenoxy)-buten-2-yl] -1-desoxynojirimycin-hydrat
N-[β-(4-Aminomethylphenoxy)-ethyl] -1-desoxynojirimycin
N-[β-(4-Hydroxymethylphenoxy)-ethyl] -1-desoxynojirimycin
N-[4-(4-Aminophenoxy)-but-2-en-yl] -1-desoxynojirimycin
N-[β-(4-Aminophenoxy)-ethyl]-1-desoxynojirimycin
N-[β-(4-Hydroxycarbonylphenoxy)-ethyl] -1-desoxynojirimycin
N-[β-(3-Hydroxycarbonylphenoxy)-ethyl] -1-desoxynojirimycin
N-[β-(4-tert.-Butylphenylthio)-ethyl] -1-desoxynojirimycin
N-(4-Phenylthiobuten-2-yl)-1-desoxynojirimycin
N-[β-(4-Cyanomethylphenoxy)-ethyl] -1-desoxynojirimycin
N-[β-(4-Aminoethylphenoxy)-ethyl] -1-desoxynojirimycin
N-[β-(4-Succinimidyloxycarbonyl)-ethyl] -1-desoxynojirimycin
N-[β-(4-Carbamoyl-phenoxy)-ethyl] -1-desoxynojirimycin
N-[β-(4-Morpholinocarbonyl-phenoxy)-ethyl] -1-desoxynojirimycin
N-[2-(4-Phenylcarbamoyl-phenoxy)-ethyl] -1-desoxynojirimycin
N-[2-(4-Phenylphenoxy)-ethyl]-1-desoxynojirimycin und
2-Hydroxymethyl-3,4,5-trihydroxy-6-methyl-piperidin,
2-Hydroxymethyl-3,4,5-trihydroxy-6-ethyl-piperidin,
2-Hydroxymethyl-3,4,5-trihydroxy-6-propyl-piperidin,
2-Hydroxymethyl-3,4,5-trihydroxy-6-n-butyl-piperidin,
2-Hydroxymethyl-3,4,5-trihydroxy-6-n-pentyl-piperidin,
2-Hydroxymethyl-3,4,5-trihydroxy-6-n-octyl-piperidin,
2-Hydroxymethyl-3,4,5-trihydroxy-6-phenyl-piperidin,

2-Hydroxymethyl-3,4,5-trihydroxy-6-n-heptyl-piperidin der D-gluco-Konfiguration und
N-Amino-1-desoxynojirimycin,
N-(Benzylidenamino)-1-desoxynojirimycin,
N-(2-Hydroxy-3-methoxybenzylidenamino)-1-desoxynojirimycin,
N-(3-Nitrobenzyliden)-amino-1-desoxyjojirimycin,
N-(3-Phenylpropyliden)-amino-1-desoxynojirimycin,
N-(4-Methylbenzyliden)-amino-1-desoxynojirimycin,
N-(4-Methoxybenzyliden)-amino-1-desoxynojirimycin,
N- (3-Cyclohexenyl-methylenamino-1-desoxynojirimycin,
N-Undecylidenamino-1-desoxynojirimycin,
N-Heptylidenamino-1-Desoxynojirimycin,
N-(Furfurylidenamino)-1-desoxynojirimycin,
N-(Thenylidenamino)-1-desoxynojirimycin,
N-(3-Pyridylmethylenamino)-1-desoxynojirimycin,
N-(4-Chlorbenzylidenamino)-1-desoxynojirimycin,
N-(2-Methylthiobenzylidenamino)-1-desoxynojirimycin,
N-(Benzylamino)-1-desoxynojirimycin,
N-(Acetamido)-1-desoxynojirimycin,
N-(Heptylamino)-1-desoxynojirimycin,
N-(Dimethylamino)-1-desoxynojirimycin,
N-(Dioctylamino)-1-desoxynojirimycin,
N-(2-Vinylsulfonylethyl)-1-desoxynojirimycin,
N-Undecen-10-yl-1-desoxynojirimycin,
1- (N′ -5-Cyano-pentylureidomethyl)-1-desoxynojirimycin,
N-(Hexa-2,4-dienyl)-1-desoxynojirimycin,
N-Methyl-1-desoxynojirimycin,
N-Butyl-1-desoxynojirimycin,
N-Ethyl-1-desoxynojirimycin,
N-Propyl-1-desoxynojirimycin,
N-Isobutyl-1-desoxynojirimycin,
N-Heptyl-1-desoxynojirimycin,
N-Benzyl-1-desoxynojirimycin,
N-(2-Pyridylmethyl)-1-desoxynojirimycin,
N- (2-Hydroxyethyl-1-desoxynojirimycin,
N-(2,3-Dihydroxypropyl)-1-desoxynojirimycin,
N-[2-(s-β-D-Glucopyranosylthio)ethyl]-1-desoxynojirimycin,
N-(2,3-Epoxypropyl)-1-desoxynojirimycin,
N-[3-Phthalimido-propyl]-1-desoxynojirimycin,
N-(3-Aminopropyl)-1-desoxynojirimycin,
N-(1-Desoxynojirimycin-yl)-essigsäure,
N-o-Nitrobenzyl-1-desoxynojirimycin,
N-o-Carboxybenzyl-1-desoxynojirimycin,
N-p-Sulfobenzyl-1-desoxynojirimycin,
N-Phenylethyl-1-desoxynojirimycin,
N-Pentyl-1-desoxynojirimycin,
N-Hexyl-1-desoxynojirimycin,
N-Octyl-1-desoxynojirimycin,
N-Nonyl-1-desoxynojirimycin,
N-Decyl-1-desoxynojirimycin,
N-Undecyl-1-desoxynojirimycin,
N-Dodecyl-1-desoxynojirimycin,
N-Tetradecyl-1-desoxynojirimycin,
N-(5-Hydroxypentyl)-1-desoxynojirimycin,
N-Cyclohexylmethyl-1-desoxynojirimycin,
N-(Cyclohex-3-enyl-methyl)-1-desoxynojirimycin,
N-(Bicyclo[2.2.2]oct-2-ylmethyl)-1-desoxynojirimycin,
N-p-Chlorbenzyl-1-desoxynojirimycin,
N-m-Methylbenzyl-1-desoxynojirimycin,

N-(4-Biphenylmethyl )-1-desoxynojirimycin,
N-(3-Phenylpropyl)-1-desoxynojirimycin,
N-Allyl-1-desoxynojirimycin,
N-(2-Propynyl)-1-desoxynojirimycin,
N-(3,4-Dichlorbenzyl)-1-desoxynojirimycin,
N-(4-Nitrobenzyl)-1-desoxynojirimycin
(N-(3-Nitrobenzyl)-1-desoxynojirimycin,
1-Cyano-1-desoxynojirimycin,
N-Methyl-1-cyano-1-desoxynojirimycin,
1-Desoxynojirimycin-1-carbonsaure,
1-Desoxynojirimycin-1-ethoxycarbonyl,
N-Methyl-1-desoxynojirimycin-1-ethoxycarbonyl,
1-Desoxynojirimycin-1-carbonsäureamid,
1-Desoxynojirimycin-1-carbonsäurebenzylamid,
N′-Methyl-1-desoxynojirimycin-1-carbonsäure-benzylamid,
1-Aminomethyl-1-desoxynojirimycin,
1-Acetylaminomethyl-1-desoxynojirimycin,
N-Methyl-1-acetylaminomethyl-1-desoxynojirimycin,
1-Benzoylaminomethyl-1-desoxynojirimycin,
N-Methyl-1-benzoylaminomethyl-1-desoxynojirimycin,
1-p-Toluolsulfonylaminomethyl-1-desoxynojirimycin,
N-Methyl-1-p-Toluolsulfonylaminomethyl-1-desoxynojirimycin,
1- (N′-Phenylureidomethyl)-1-desoxynojirimycin,
N-(1-Desoxynojirimycin-yl)-essigsäure-6-lacton,
N-(1-Desoxynojirimycin-yl)-essigsäurebenzylamid,
N-(1-Desoxynojirimycin-yl)-essigsäure-butylamid,
1-Hydroxymethyl-1-desoxynojirimycin,
6-O-Benzoyl-1-desoxynojirimycin,
N-(2-Methoxyethyl)-1-desoxynojirimycin,
N-(2-Methylthioethyl)-1-desoxynojirimycin,
N-(2-Ethylthio)-1-desoxynojirimycin,
N-[2-(2-Methoxyethoxy)-ethyl]-1-desoxynojirimycin,
N-Nonyl-1-acetylaminomethyl-1-desoxynojirimycin,
1-Nonylaminomethyl-1-desoxynojirimycin,
N-Phenyl-1-desoxynojirimycin,
N-Cyclohexyl-1-desoxynojirimycin,
N-Isopropyl-1-desoxynojirimycin,
N-(1-Desoxyglucityl)-1-desoxynojirimycin,
N-(2-Phenoxyethyl)-1-desoxynojirimycin,
N-(5-Phenoxypentyl)-1-desoxynojirimycin,
N-(4-Phenoxybutyl-1-desoxynojirimycin,
N-(2-(2,6-Xylyloxy)ethyl)-1-desoxynojirimycin,
N-(3-(2,6-Xylyloxy)propyl)-1-desoxynojirimycin,
N-(2-(2,4-Dichlorphenoxy)ethyl-1-desoxynojirimycin,
N-(4-Phenoxy-2-butenyl)-1-desoxynojirimycin,
N-[4-(4-Tolyloxy)-2-butenyl]-1-desoxynojirimycin,
N-[4-(4-Ethoxycarbonylphenoxy)-2-butenyl]-1-desoxynojirimycin,
N-[2-(4-Methoxyphenoxy)ethyl]-1-desoxynojirimycin,
N-[2-(4-Chlorphenoxy)ethyl]-1-desoxynojirimycin,
N[2-(4-Cyanophenoxy)ethyl]-1-desoxynojirimycin,
N-[2-(3-Tolyloxy)ethyl)]-1-desoxynojirimycin,
N-(2-Phenylthioethyl)-1-desoxynojirimycin,
N-[2-(4-Tolylthio)ethyl]-l-desoxynojirimycin,
N-[4-(3-Tolylthio)-2-butenyl]-1-desoxynojirimycin,
N-[4-(4-Chlorphenylthio)-2-butenyl-1-desoxynojirimycin,
N[4(tert.-Butylphenylthio)-2-butenyl]-1-desoxynojirimycin,
N-[4-(4-Tolylthio)-2-butenyl]-1-desoxynojirimycin,
N-[4-(4-Biphenyloxy)-2-butenyl]-1-desoxynojirimycin,

N-(3-Butenyl)-1-desoxynojirimycin,
N-(2,4-Hexadienyl)-1-desoxynojirimycin,
N-(2,4-Heptadienyl)-1-desoxynojirimycin,
N-Cinnamyl-1-desoxynojirimycin,
N-(2-Pentenyl)-1-desoxynojirimycin,
N-(2-Butenyl)-1-desoxynojirimycin,
N-(8,8-Dimethyl-2-nonenyl)-1-desoxynojirimycin.

Es wurde im Rahmen von Untersuchungen, die zu der vorliegenden Erfindung führten, überraschend gefunden, daß die oben genannten Verbindungen der allgemeinen Formel (I) antiviral, und zwar gegen Retroviren wirksam sind, Dies wird durch die weiter unter angegebenen Versuchsdaten beispielhaft für die Verbindungen am Visnavirus in Zellkultur belegt. Das Visna-Virus und das HIV-Virus (Virus der humanen Immunodefiziens) gehören beide zu der Retrovirus-Subfamilie der Lentiviren (Haase A.T. Nature (1986) 322, 130 - 136). Beide Viren weisen eine ähnliche Genomorganisation und ein gegenüber den anderen Retroviren komplexes Transpkriptionsmuster auf (sonijo P. et. al, Cell (1985), 42, 369 - 382; Davis J. L. et. al. Journal of Virology (1987) 61 (5) 1325 - 1331.

In Zellkulturen, die mit Visna-Virus infiziert sind, treten 5 bis 10 Tage nach der Infektion ausgeprägte, virusinduzierte, zytopathische Effekte auf. Durch Behandlung der infizierten Zellkulturen mit den erfindungsgemäßen Verbindungen konnte das Auftreten dieser zytopathischen Effekte verhindert werden. Da sie das Glykosylierungsmuster der Virusproteine beeinflussen, können die erfindungsgemäßen Verbindungen zur Herstellung abgeschwächter oder nicht infektiöser Viruspartikel verwendet werden, die ihrerseits zur Vakineherstellung eingesetzt werden können. Der Visna-VirusTest wurde nach der methode von O. Narayan et. al., Journal of Infektious Diseases 135, 5, (1977), 800 - 806 durchgeführt. Dazu wurde die erfindungsgemäße Verbindungen im Kulturmedium in nicht zytotoxischen Konzentrationen in 96er Microtiterplatten verdünnt. Anschließend wurden Fibroblastenzellen vom Schaf (5 x $10^4$ Zellen pro Napf) in Produktionsmedium zu jedem Näpfchen zugeführt. Jedes Näpfchen erhielt dann 50 µl einer Visna-Viruslösung mit einem Titer von ca. 2,5 x $10^4$ $TCID_{50}$ (TCIC = tissue culture infectious dosis).

Diese Virusdosis entspricht eine MOI (Multiplizität der Infektion) von ca. 0,05.

Unter diesen Infektionsbedingungen resultierte zwischen Tag 5 und Tag 10 in der Infektionskontrolle ohne Testsubstanz ein virusinduzierter, zytopathischer Effekt. Die infizierten und behandelten Zellen und die Kontrollzellen wurden 7 - 9 Tage bei 37° C, 5 %, $CO_2$, inkubiert.

Bei Auftreten des virusinduzierten, zytopathogenen Effektes in der unbehandelten Viruskontrolle wurden die Kulturen mit Formalin fixiert und anschließend mit einer Giemsa-Lösung gefärbt. Die inhibitorische Konzentration ($IC_{50}$) wurde mikroskopisch als die Konzentration ermittelt, bei der der zytopathische Effekt um 50 % gehemmt wurde im Vergleich zur unbehandelten Viruskontrolle, die 100 % Zellzerstörung aufwies.

Es wurde gefunden, daß beispielsweise H-(2-Butenyl)-1-desoxynojirimycin in einem Konzentrationsbereich von 500 µg/ml bis 30 µg/ml die mit Visna-Virus infizierten Zellen vor der virusinduzierten Zellzerstörung schützen.

Gleiche schützende Effekte konnten beobachtet werden bei der Anwendung von N-(Prop-1-en-3yl )-1-desoxynojirimycin und N-(1-Phenyl-prop-1en-3yl-1-desoxynojirimycin wie in Tabelle 1 dargestellt.

| Verbindung Nr. | $Ic_{50}$ /µg/ml |
|---|---|
| 1 | 30 |
| 2 | 100 |
| 3 | 50 |
| 4 | 250 |
| 5 | 50 |
| 6 | 30 |
| 7 | 10 |
| 8 | 10 |
| 9 | 10 |
| 10 | 20 |

1 N-(2-Butenyl)-1-desoxynojirimycin
2 N-(4-(4-Methylphenylthio)-buten-2yl)-1-desoxynojirimycin
3 N-Amino-1-desoxynojirimycin
4 2-Hydroxymethyl-3,4,5-trihydroxy-6-butylpiperidine
5 N-(Prop-1-en-3yl)-1-desoxynojirimycin
6 N-(1-Phenyl-prop-1en-3yl)-1-desoxynojirimycin
7 N-Ethyl-1-desoxynojirimycin
8 N-Methyl-1-desoxynojirimycin
9 N-Butyl-1-desoxynojirimycin
10 N-Propyl-1-desoxynojirimycin

Die erfindungsgemäß zu verwendenden Verbindungen stellen somit wertvolle Wirkstoffe zu Behandlung und Prophylaxe von Erkrankungen, hervorgerufen durch Retroviren, in der Human- und Tiermedizin dar.

Als Indikationsgebiete in der Humanmedizin können beispielsweise genannt werden:

1.) Die Behandlung oder Prophylaxe von menschlichen Retrovirusinfektionen.

2.) Für die Behandlung oder Prophylaxe von HIV (Virus der humanen Immundefiziens; früher HTLV III/LAV genannt) verursachen Erkankungen (AIDS) und den damit assozierten Stadien wie ARC (AIDS related complex) und LAS (Lymphadenopathie-Syndrom) sowie der durch dieses Virus verursachten Immunschwäche und Encephalopathie.

3.) Für die Behandlung oder die Prophylaxe einer HTLV I- oder HTLV II-Infektion.

4.) Für die Behandlung oder die Prophylaxe des AIDS-carrier Zustandes (AIDS-Überträger-Zustand).

Als Indikationen in der Tiermedizin können beispielsweise angeführt werden:

Infektionen mit

a) Maedivisna (bei Schafen und Ziegen)

b) progressives Pneumonievirus (PPV) (bei Schafen und Ziegen)

c) caprine arthritis encephalitis Virus (bei Schafen und Ziegen)

d) Zwoegerziekte Virus (bei Schafen)
e) infektiöses Virus der Anämie (des Pferdes)
f) Infektionen verursacht durch das T-Zellen-Katzenleukämievirus

Zur vorliegenden Erfindung gehören pharmazeutische Zubereitungen, die neben nicht-toxischen, inerten pharmazeutisch geeigneten Trägerstoffen eine oder mehrere Verbindungen der Formel (I) bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetyl-alkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dme oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol Isopropylalkohol Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykol und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffen wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusol und Süßmittel, z.B. Saccharin,

enthalten,

Die Wirkstoffe der Formel (I) sollen in den oben aufgeführten pharmazeutischen Zubereitungen vorzugsweise in einer Konzentration von etwa 0,1 bis 99,5, vorzugsweise von etwa 0,5 bis 95 Gew,-%, der Gesamtmischung vorhanden sein.

Die oben aufgeführten pharmazeutischen Zubereitungen können außer den Verbindungen der Formel (I) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, Lokal (Puder, Salbe, Tropfen) und zur Therapie von Infektionen in Hohlräumen, Körperhöhlen angewendet werden, Als geeignete Zubereitungen kommen Injektionslösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden, Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Tabletten, Boli, Kapseln, Aerosole, Sprays, Inhalate bei Mensch und Tier angewendet werden, Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die Wirkstoffe der Formel (I) in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw, Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß, Die Festlegung der jeweils erforderlichen optimalen Dosierung und Applikationsart der Wirkstoffe kann durch jeden Fachmann aufgrund seines Fachwissens leicht erfolgen.

Die erfindungsgemäß zu verwendenden Verbindungen können in den üblichen Konzentrationen und Zubereitungen zusammen mit dem Futter bzw, mit Futterzubereitungen oder mit den Trinkwasser gegeben werden.

## Ansprüche

1. Verwendung von 2-Hydroxymethylen-3,4,5-trihydroxypiperidinen oder eines pharmazeutisch anwendbaren Salzes davon der allgemeinen Formel (I)

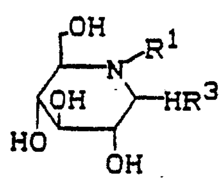

(I)

in welcher

$R^1$ für Wasserstoff steht und

$R^3$ für einen gegebenenfalls substituierten geradkettigen, verzweigten oder cyclischen gesättigten oder ungesättigten, gegebenenfalls durch Heteroatome wie O oder S, unterbrochenen Kohlenwasserstoffrest, einen gegebenenfalls substituierten aromatischen Rest oder einen gegebenenfalls substituierten heterocyclischen Rest steht.

oder in welcher

$R^1$ für Wasserstoff oder für einen gegebenenfalls substituierten, geradkettigen, verzweigten oder cyclischen,

gesättigten oder ungesättigten Kohlenwasserstoffrest, oder für einen gegebenenfalls substituierten aromatischen oder heterocyclischen Rest steht
und
R3 für -H, OH, $-OR^5$, -SH, $SR^5$, $-NH_2$, $-NHR^5$,

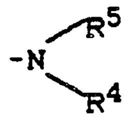,

$NH_2CH_2$-, $NHR^5$-$CH_2$-, $NR^5R^4$-$CH_2$-, -COOH, $-COOR^5$, HO-$CH_2$-, $R^5CO$-$NHCH_2$-, $R^5CO$-$NR^4CH_2$-, $R^5SO_2NHCH_2$-, $R^5SO_2$-$NR^4CH_2$-,
$R^5-NH-\underset{\underset{O}{\|}}{C}-NH-CH_2-$, $R^5-NH-\underset{\underset{S}{\|}}{C}-NH-CH_2-$,
$R^5-O-\underset{\underset{O}{\|}}{C}-NH-CH_2-$,
$-SO_3H$, -CN, $-CONH_2$, $CONHR^5$ oder $CONR^5R^4$ steht,
wobei
$R^5$, $R^4$ die vorstehend für $R^1$ genannten Bedeutungen annehmen konnen,
und wobei für den Fall $R^3 = H$ oder OH, $R^1$ für einen gegebenenfalls substituierten, geradkettigen, verzweigten oder cyclischen, gesättigten oder ungesättigten Kohlenwasserstoffrest oder für einen gegebenenfalls substituierten aromatischen oder heterocyclischen Rest steht,
oder in welcher
$R^1$ für

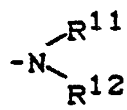

steht
worin
$R^{11}$ Wasserstoff, -Formyl, $-R^{14}$, $-COR^{14}$, $CO_2R^{14}$, $CONH_2$, $CONHR^{14}$, $CONR^{14}R^{15}$, $CSR^{14}$, $CSNH_2$, $CSNHR^{14}$, $CSNR^{14}R^{15}$, $SO_3H$ oder $SO_2R^{14}$,
$R^{12}$ Wasserstoff oder $R^{14}$ oder
$R^{11}$ und $R^{12}$ gemeinsam die Gruppierung

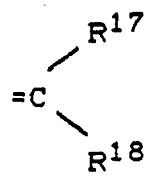

$R^3$ Wasserstoff, Hydroxy, $OR^{14}$, Mercapto, $SR^{14}$, Amino, $NHR^{14}$, $NR^{14}R^{15}$, Cyano, Carboxy, $CO_2R^{14}$, Carboxylamido, $CONHR^{14}$, $CONR^{14}R^{15}$, Aminomethyl, $CH_2NHR^{14}$, $CH_2NR^{14}R^{15}$, $CH_2$-$NR^{14}COR^{15}$, $CH_2NR^{14}SO_2R^{15}$, Sulfo, Hydroxymethyl, $CH_2OR^{14}$ oder $CH_2OCOR^{14}$,
bedeuten, und
$R^{14}$, $R^{15}$ unabhängig voneinander einen gegebenenfalls substituierten, geradkettigen, verzweigten oder cyclischen gesättigten oder ungesättigten aliphatischen Kohlenwasserstoffrest oder einen gegebenenfalls substituierten aromatischen oder heterocyclischen Rest, wobei 2 Reste $R^{15}$, $R^{17}$ und $R^{18}$ auch miteinander verknüpft sein können, bedeuten, und
$R^{17}$ und $R^{18}$ unabhängig voneinander Wasserstoff oder $R^{15}$ bedeuten,
oder in welcher
$R^3$ für Wasserstoff und
$R^1$ für

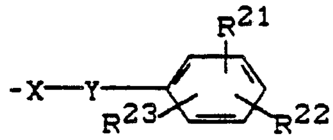

steht,
worin
X für einen geradkettigen oder verzweigten gesättigten oder ungesättigten Kohlenwasserstoffrest steht,
Y für Sauerstoff oder Schwefel steht und
$R^{21}$, $R^{22}$, $R^{23}$ gleich oder verschieden sind und unabhängig voneinander für Wasserstoff, Halogen, Alkyl, Aryl, Alkoxy, Aroxy, Alkylthio, Nitro, Amino, Hydroxy, Cyano, Alkyl- und Dialkylamino, Acylamino, Acyloxy, Carboxyl, Carbalkoxy, Alkylcarbonyl, Formyl oder einen gegebenenfalls substituierten Sulfonamidrest stehen,
bei der Bekämpfung von viralen Erkrankungen

2. Verwendung von 2-Hydroxymethylen-3,4,5-trihydroxypiperidinen aus der Gruppe
N-(2-Vinylsulfonylethyl)-1-desoxynojirimycin,
N-Undecen-10-yl-1-desoxynojirimycin,
1-(N′-5-Cyano-pentylureidomethyl )-1desoxynojirimycin,
N-(Hexa-2,4-dienyl)-1-desoxynojirimycin,
N-Methyl-1-desoxynojirimycin,
N-Butyl-1-desoxynojirimycin,
N-Ethyl-1-desoxynojirimycin,
N-Propyl-1-desoxynojirimycin,
N-Isobutyl-1-desoxynojirimycin,
N-Heptyl-1-desoxynojirimycin,
N-Benzyl-1-desoxynojirimycin,
N-(2-Pyridylmethyl)-1-desoxynojirimycin,
N-(2-Hydroxyethyl)-1-desoxynojirimycin,
N-(2,3-Dihydroxypropyl)-1-desoxynojirimycin,
N[2-(s-β-D-Glucopyranosylthio)ethyl]-1-desoxynojirimycin,
N-(2,3-Epoxypropyl)-1-desoxynojirimycin,
N-[3-Phthalimido-propyl]-1-desoxynojirimycin,
N-(3-Aminopropyl-1-desoxynojirimycin,
N-(1-Desoxynojirimycin-yl)-essigsäure,
N-o-Nitrobenzyl-1-desoxynojirimycin,
N-o-Carboxybenzyl-1-desoxynojirimycin,
N-p-Sulfobenzyl-1-desoxynojirimycin,
N-Phenylethyl-1-desoxynojirimycin,
N-Pentyl-1-desoxynojirimycin,
N-Hexyl-1-desoxynojirimycin,
N-Octyl-1-desoxynojirimycin,
N-Nonyl-1-desoxynojirimycin,
N-(3-Butenyl)-1-desoxynojirimycin,
N-(2,4-Hexydienyl)-1-desoxynojirimycin,
N-(2,4-Heptadienyl)-1-desoxynojirimycin,
N-Cinnamyl-1-desoxynojirimycin,
N-Decyl-1-desoxynojirimycin,
N-Undecyl-1-desoxynojirimycin,
N-Dodecyl-1-desoxynojirimycin,
N-Tetradecyl-1-desoxynojirimycin,
N-(5-Hydroxypentyl)-1-desoxynojirimycin,
N-Cyclohexylmethyl-1-desoxynojirimycin,
N-(Cyclohex-3-enyl-methyl)-1-desoxynojirimycin,
N-(Bicyclo[2.2.2]oct-2-ylmethyl)-1-desoxynojirimycin,
N-p-Chlorbenzyl-1-desoxynojirimycin,
N-m-Methylbenzyl-1-desoxynojirimycin,

N-(4-Biphenylmethyl )-1-desoxynojirimycin,
N-(3-Phenylpropyl)-1-desoxynojirimycin,
N-Allyl-1-desoxynojirimycin,
N-(2-Propyl)-1-desoxynojirimycin,
N-(3,4-Dichlorbenzyl)-1-desoxynojirimycin,
N-(4-Nitrobenzyl)-1-desoxynojirimycin
(N-(3-Nitrobenzyl)-1-desoxynojirimycin,
1-Cyano-1-desoxynojirimycin,
N-Methyl-1-cyano-1-desoxynojirimycin,
1-Desoxynojirimycin-1-carbonsaure,
1-Desoxynojirimycin-1-ethoxycarbonyl,
N-Methyl-1-desoxynojirimycin-1-ethoxycarbonyl,
1-Desoxynojirimycin-1-carbonsäureamid,
1-Desoxynojirimycin-1-carbonsäurebenzylamid,
N-Methyl-1-desoxynojirimycin-1-carbonsäure-benzylamid,
1-Aminomethyl-1-desoxynojirimycin,
1-Acetylaminomethyl-1-desoxynojirimycin,
N-Methyl-1-acetylaminomethyl-1-desoxynojirimycin,
1-Benzoylaminomethyl-1-desoxynojirimycin,
N-Methyl-1-benzoylaminomethyl-1-desoxynojirimycin,
1-p-Toluolsulfonylaminomethyl-1-desoxynojirimycin,
N-Methyl-1-p-Toluolsulfonylaminomethyl-1-desoxynojirimycin,
1- (N′-Phenylureidomethyl)-1-desoxynojirimycin,
N-(1-Desoxynojirimycin-yl)-essigsäure-6-lacton,
N-(1-Desoxynojirimycin-yl)-essigsäurebenzylamid,
N-(1-Desoxynojirimycin-yl)-essigsäure-butylamid,
1-Hydroxymethyl-1-desoxynojirimycin,
6-O-Benzoyl-1-desoxynojirimycin,
N-(2-Methoxyethyl)-1-desoxynojirimycin,
N-(2-Methylthioethyl)-1-desoxynojirimycin,
N-(2-Ethylthio)-1-desoxynojirimycin,
N-[2-(2-Methoxyethoxy)-ethyl]-1-desoxynojirimycin,
N-Nonyl-1-acetylaminomethyl-1-desoxynojirimycin,
1-Nonylaminomethyl-1-desoxynojirimycin,
N-Phenyl-1-desoxynojirimycin,
N-Cyclohexyl-1-desoxynojirimycin,
N-Isopropyl-1-desoxynojirimycin,
N-(1-Desoxyglucityl)-1-desoxynojirimycin,
N-(2-Phenoxyethyl)-1-desoxynojirimycin,
N-(5-Phenoxypentyl)-1-desoxynojirimycin,
N-(4-Phenoxybutyl-1-desoxynojirimycin,
N-(2-(2,6-Xylyloxy)ethyl)-1-desoxynojirimycin,
N-(3-(2,6-Xylyloxy)propyl)-1-desoxynojirimycin,
N-(2-(2,4-Dichlorphenoxy)ethyl-1-desoxynojirimycin,
N-(4-Phenoxy-2-butenyl)-1-desoxynojirimycin,
N-[4-(4-Tolyloxy)-2-butenyl]-1-desoxynojirimycin,
N-[4-(4-Ethoxycarbonylphenoxy)-2-butenyl]-1-desoxynojirimycin,
N-[2-(4-Methoxyphenoxy)ethyl]-1-desoxynojirimycin,
N-[2-(4-Chlorphenoxy)ethyl]-1-desoxynojirimycin,
N-[2-(4-Cyanophenoxy)ethyl]-1-desoxynojirimycin,
N-[2-(3-Tolyloxy)ethyl)-1-desoxynojirimycin,
N-(2-Phenylthioethyl)-1-desoxynojirimycin,
N-[2-(4-Tolylthio)ethyl)-1-desoxynojirimycin,
N-[4-(3-Tolylthio)-2-butenyl]-1-desoxynojirimycin,
N-[4-(4-Chlorphenylthio)-2-butenyl]-1-desoxynojirimycin,
N-[4-(4-Tolylthio)-2-butenyl]-1-desoxynojirimycin,
N-[4-(4-Biphenylyloxy)-2-butenyl]-1-desoxynojirimycin,
N-(2-Pentenyl)-1-desoxynojirimycin,

N-(2-Butenyl)-1-desoxynojirimycin
N-(8,8-Dimethyl-2-nonenyl)-1-desoxynojirimycin
N-β-Phenoxyethyl-1-desoxynojirimycin
N-(5-Phenoxy-pentyl)-1-desoxynojirimycin
N- (4-Phenoxy-butyl)-1-desoxynojirimycin
N-[β-(2,6-Dimethyl-phenoxy)-ethyl]-1-desoxynojirimycin
N-[γ-(2,6-Dimethoxyphenoxy)-propyl]-1-desoxynojirimycin
N-[β-(2,4-Dichlorphenoxy)-ethyl]-1-desoxynojirimycin
N-(γ-Phenoxypropyl)-1-desoxynojirimycin
N-(4-Phenoxy-trans-buten-2-yl-1-desoxynojirimycinhydrat
H-(p-Methoxyphenyloxy-trans-buten-2-yl)-1desoxynojirimycin
N-[(4-Carbethoxyphenyloxy)-buten-2-yl]-1-desoxynojirimycin
N-β-(4-Methoxy-phenoxy)-ethyl]-1-desoxynojirimycin
N-[β-(4-Carbethoxyphenyloxy)-ethyl]-1-desoxynojirimycin
N-[β-(4-Cyano-phenoxy)-ethyl]-1-desoxynojirimycin
N-β- (3-Methylphenoxy)-ethyl]-1-desoxynojirimycin
N-(β-Phenylthioethyl)-1-desoxynojirimycin
N-[β-(4-Methylphenylthio)-ethyll-1-desoxynojirimycin
N-[4-(4-Methylphenylthio)-buten-2-yl]-1-desoxynojirimycin
N-[4-(4-Chlorphenylthio)-buten-2-yl]-1-desoxynojirimycin
N-[4-(4-tert.-Butylphenylthio)-buten-2-yl] -1-desoxynojirimycin
N-[4-(4-Methylphenylthio)-buten-2-yl] -1-desoxynojirimycin
N-[4-(4-Phenylphenoxy)-buten-2-yl]-1-desoxynojirimycin
N-[β-(4-Acetamidophenoxy)-ethyl]-1-desoxynojirimycin
N-β-(4-Ethoxycarbonyl-phenoxy)-ethyl] -1-desoxynojirimycin
N-[β-(4-Formylphenoxy)-ethyl]-1-desoxynojirimycin
N-[β-(4-Hydroxyphenoxy)-ethyl]-1-desoxynojirimycin
N-[β-(3-Ethoxycarbonylphenoxy)-ethyl] -desoxynojirimycin
N-[4-(4-Acetamidophenoxy)-buten-2-yl] -1-desoxynojirimycin-hydrat
N-[β-(4-Aminomethylphenoxy)-ethyl] -1-desoxynojirimycin
N-[β-(4-Hydroxymethylphenoxy)-ethyl] -1-desoxynojirimycin
N-[β-(4-Aminophenoxy)-ethyl]-1-desoxynojirimycin
N-[β-(4-Hydroxycarbonylphenoxy)-ethyl] -1-desoxynojirimycin
N-[β-(3-Hydroxycarbonylphenoxy)-ethyl] -1-desoxynojirimycin
N-[β-(-(4-tert.-Butylphenylthio)-ethyl] -1-desoxynojirimycin
N-(4-Phenylthiobuten-2-yl)-1-desoxynojirimycin
N-[β-(4-Cyanomethylphenoxy)-ethyl] -1-desoxynojirimycin
N-[β-(4-Aminoethylphenoxy)-ethyl] -1-desoxynojirimycin
N-[β-(4-Succinimidyloxycarbonyl)-ethyl] -1-desoxynojirimycin
N-[β-(4-Carbamoyl-phenoxy-ethyl] -1-desoxynojirimycin
N-[β-(4-Morpholinocarbonyl-phenoxy)-ethyl] -1-desoxynojirimycin
N-[2-(4-Phenylcarbamoyl-phenoxy)-ethyl] -1-desoxynojirimycin und
N-[2-(4-Phenylphenoxy)-ethyl]-1-desoxynojirimycin
N-Amino-1-desoxynojirimycin,
N-(Benzylidenamino)-1-desoxynojirimycin,
N-(2-Hydroxy-3-methoxybenzylidenamino)-1-desoxynojirimycin,
N-(3-Nitrobenzyliden)-amino-1-desoxynojirimycin,
N-(3-Phenylpropyliden)-amino-1-desoxynojirimycin,
N-(4-Methylbenzyliden)-amino-1-desoxynojirimycin,
N-(4-Methoxybenzyliden)-amino-1-desoxynojirimycin,
N-(3-Cyclohexenyl)-methylenamino-1-desoxynojirimycin,
N-Undecylidenamino-1-desoxynojirimycin,
N-Heptylidenamino-1-Desoxynojirimycin,
N-(Furfurylidenamino)-1-desoxynojirimycin,
N-(Thenylidenamino)-1-desoxynojirimycin,
N-(3-Pyridylmethylenamino)-1-desoxynojirimycin,
N-(4-Chlorbenzylidenamino)-1-desoxynojirimycin,
N-(2-Methylthiobenzylidenamino)-1-desoxynojirimycin,

N-(Benzylamino)-1-desoxynojirimycin,
N-(Acetamido)-1-desoxynojirimycin,
N-(Heptylamino)-1-desoxynojirimycin,
N-(Dimethylamino)-1-desoxynojirimycin
N-(Dioctylamino)-1-desoxynojirimycin
N-Hydroxmethyl-3,4,5-trihydroxy-6-methylpiperidin,
2-Hydroxymethyl-3,4,5-trihydroxy-6-ethylpiperidin,
2-Hydroxmethyl-3,4,5-trihydroxy-6-propyl-piperidin,
2-Hydroxymethyl-3,4,5-trihydroxy-6-n-butyl-piperidin,
2-Hydroxymethyl-3,4,5-trihydroxy-6-n-pentyl-piperidin,
2-Hydroxymethyl-3,4,5-trihydroxy-6-n-octyl-piperidin,
2-Hydroxmethyl-3,4,5-trihydroxy6-phenyl-piperidin oder
2-Hydroxymethyl-3,4,5-trihydroxy-6-n-heptyl-piperidin
der D-gluco-Konfiguration bei der Bekämpfung von viralen Erkrankungen.

3. Verwendung von Hydroxypiperidinen aus den Ansprüchen 1 und 2 zur Bekämpfung von retroviralen Infektionen.

4. Verwendung von Hydroxypiperidinen aus den Ansprüchen 1 und 2 bei der Herstellung von Arzneimitteln mit antiviraler Wirkung.

5. Verwendung von Hydroxypiperidinen aus den Ansprüchen 1 und 2 bei der Herstellung von Arzneimitteln mit antiretroviraler Wirkung.